# EUROPEAN PATENT SPECIFICATION

(11) **EP 3 965 696 B1**
(45) Date of publication and mention of the grant of the patent: **10.12.2025**
(21) Application number: 20801996.8
(22) Date of filing: 08.05.2020
(51) Int. Cl.: A61F 2/24, A61F 2/00, A61F 2/02, A61F 2/95, A61F 2/962

(54) **INVERTED DELIVERY TECHNIQUES FOR PROSTHETIC HEART VALVES**
UMGEKEHRTE FREISETZUNGSTECHNIKEN FÜR HERZKLAPPENPROTHESEN
TECHNIQUES DE POSE INVERSÉE DE VALVULES CARDIAQUES PROTHÉTIQUES

(30) Priority: 09.05.2019 US 201962845857 P
(43) Date of publication of application: 16.03.2022
(73) Proprietor: Caisson Interventional, LLC, Maple Grove, Minnesota 55311 (US)
(72) Inventor: MONTAG, Benjamin, Delano, Minnesota 55328 (US); SCHNEIDER, Lucas, Champlin, Minnesota 55316 (US); BAHOORA, Kim, Maple Grove, Minnesota 55311 (US); GARVEY, Zachary, Maple Grove, Minnesota 55311 (US); MORTIER, Todd, Mound, Minnesota 55364 (US); SCHWEICH, Cyril, Maple Grove, Minnesota 55311 (US); GANESAN, Kavitha, Minnetrista, Minnesota 55364 (US); IYER, Ramji, Maple Grove, Minnesota 55311 (US)
(74) Representative: Grünecker Patent- und Rechtsanwälte PartG mbB
(86) International application number: PCT/US2020/032230
(87) International publication number: WO 2020/227685

(56) References cited:
- EP-A1- 2 695 586
- WO-A1-2013/072496
- WO-A1-2018/178967
- US-A1- 2011 137 397
- US-A1- 2015 112 433
- US-A1- 2015 351 904

## Description

### TECHNICAL FIELD

The present disclosure relates to heart valve interventional systems and methods and, more particularly, to methods for delivering and repositioning mitral valves.

### BACKGROUND

Human heart valves, which include the aortic, pulmonary, mitral and tricuspid valves, function in synchronization with the pumping heart to control the flow of blood between chambers of the heart. In short, the valves allow blood to flow downstream and inhibit blood from flowing upstream. Diseased heart valves exhibit impairments such as narrowing of the valve, remodeling of the annulus, or calcification, which inhibit the valves from properly controlling blood flow. Such impairments reduce the heart's blood-pumping efficiency and can be a debilitating or, in some situations, life threatening. Thus, extensive efforts have been made to develop methods and devices to repair or replace impaired heart valves.

One technique for addressing a damaged or defective heart valve is to replace the native valve with a valve prosthesis. One category of heart valve prosthesis includes those that can be delivered in a minimally invasive fashion so as to minimize trauma to the patient. Replacement valves are being designed to be delivered through minimally invasive procedures and even percutaneous procedures. Such replacement valves often include a tissue-based valve that is connected to an expandable frame that is then delivered to the native valve's annulus.

Development of prostheses including but not limited to replacement heart valves that can be collapsed for minimally invasive delivery and then controllably expanded has proven to be particularly challenging. An additional challenge relates to the ability of such prostheses to be secured relative to adjacent native tissue. Adequate anchoring of the prosthesis is important to ensuring the successful operation of the prosthetic heart valve for a sufficient length of time. WO 2013/072496 relates to an implant designed to be placed in a blood flow passage, the implant being deployable between a contracted configuration and a deployed configuration, comprising a tubular frame with a central axis defining an inner blood flow conduit and a plurality of distal arms extending perpendicular to the central axis in the deployed configuration to press on a first face of the tissue and a plurality of proximal arms having an end connected to the frame and a free end designed to press on a second face of the tissue to clamp the tissue.

### SUMMARY

The present invention concerns a prosthetic device for implanting at a native mitral or tricuspid valve region of the heart as further defined by claim 1. The present disclosure describes shapes and geometries of anchoring elements, including feet, used to secure a prosthetic valve, e.g., a prosthetic mitral valve, in the native heart valve annular position. In embodiments, the foot geometry/directionality is designed such that the foot loads to the fibrous annular region that is made of collagenous tissue with higher puncture resistance. Additionally, the foot contact surface area is designed such that the pressure exerted by the foot is below the pressure required to puncture the heart tissue, such as the left ventricular (LV) muscle wall. Further, the present disclosure provides an improved method of delivering and/or repositioning a valve dock and/or valve replacement system by inverting the implant anchors, e.g., including the feet, to point downward, i.e., toward the ventricle, while crossing the native mitral valve annulus. The valve feet are then deployed once the tips of the feet are a sufficient distance past the annulus. To reposition the valve, the frame is retracted using delivery mechanisms to return the feet to the inverted state, which can be used for removing and/or reattempting deployment of the valve.

As recited in examples, Example 1 is a method of delivering a valve prosthesis to be deployed within a native heart valve at a native heart valve annulus. The method including collapsing a prosthetic valve having an expandable frame comprising a proximal end and a distal end and a longitudinal axis extending therethrough inside of a delivery instrument, such that a plurality of spaced anchors extending from the distal end of the expandable frame towards the proximal end are collapsed to an inverted position in a collapsed anchor configuration. The method further including advancing the delivery instrument a first distance such that the plurality of spaced anchors extend below the valve annulus, and releasing each of the plurality of spaced anchors such that each of the plurality of spaced anchors is repositioned from the inverted position to an expanded anchor configuration to contact a subannular location

Example 2 is the method of Example 1, comprising adjusting an angle of each of the plurality of spaced anchors, such that upon release the spaced anchors contact the subannular location.

Example 3 is the method of Example 1, where releasing each of the plurality of spaced anchors includes releasing each of the plurality of spaced anchors such that each of the plurality of spaced anchors has a foot angle of from 0 to 45 degrees relative to the longitudinal axis.

Example 4 is the method of Example 1, wherein collapsing the prosthetic valve includes collapsing the plurality of spaced anchors to the collapsed anchor configuration, such that each of the plurality of spaced anchors pivots to an outward and progressively downward position.

Example 5 is the method of Example 1, wherein collapsing the prosthetic valve includes collapsing the plurality of spaced anchors such that each of the plurality of spaced anchors pivots from a foot angle of from 0 to 45 degrees to an outward and downward foot angle of from 45 to 180 degrees.

Example 6 is the method of Example 1, wherein the delivery instrument includes an outer catheter member attached to the expandable frame adjacent the proximal end and an inner catheter member attached to the expandable frame adjacent the distal end.

Example 7 is the method of Example 6, wherein collapsing the prosthetic valve includes retracting the inner catheter member into the outer catheter member to pivot each of the plurality of spaced anchors to an outward and progressively downward position.

Example 8 is the method of Example 6, wherein releasing each of the plurality of spaced anchors includes extending the inner catheter member out of the outer catheter member to pivot each of the plurality of spaced anchors to the expanded anchor configuration.

Example 9 is the method of Example 1, wherein each of the plurality of spaced anchors are locked into the collapsed anchor configuration prior to advancing the delivery instrument.

Example 10 is the method of Example 1, comprising at least one of repositioning and removing the valve prosthesis by collapsing the prosthetic valve, such that each of the plurality of spaced anchors collapses to the inverted position.

Example 11 a method of delivering a valve prosthesis to be deployed within a native heart valve at a native heart valve annulus. The method including collapsing a prosthetic valve having an expandable frame comprising a proximal end and a distal end and a longitudinal axis extending therethrough inside of a delivery instrument, such that a plurality of spaced anchors extending from the distal end of the expandable frame towards the proximal end pivot outward and downward from an expanded foot angle of from 0 to 45 degrees to a collapsed foot angle of from 45 to 180 degrees. The method further including advancing the delivery instrument such that the plurality of anchors extend below the valve annulus, and releasing each of the plurality of anchors such that each of the plurality of anchors pivots to the expanded foot angle of from 0 to 45 degrees.

Example 12 is the method of Example 11, where releasing each of the plurality of anchors repositions the anchors for contacting subannular tissue.

Example 13 is the method of Example 11, wherein the delivery instrument includes an outer catheter member attached to the expandable frame adjacent the proximal end and an inner catheter member attached to the expandable frame adjacent the distal end.

Example 14 is the method of Example 13, wherein collapsing the prosthetic valve includes retracting the inner catheter member into the outer catheter member to pivot each of the plurality of anchors to an inverted position.

Example 15 is the method of Example 13, wherein releasing each of the plurality of anchors includes extending the inner catheter member out of the outer catheter member to pivot each of the plurality of anchors to the expanded foot angle for contacting subannular tissue.

Example 16 is a valve prosthesis system including a prosthetic valve and a delivery instrument according to the invention. The prosthetic valve having an expandable frame including a proximal end and a distal end and a longitudinal axis extending therethrough. The expandable frame configured to collapse radially for delivery and expand radially upon deployment to an expanded configuration. The prosthetic valve including a plurality of anchors extending from the distal end of the expandable frame towards the proximal end, each anchor being expandable from a collapsed anchor configuration to an expanded anchor configuration. The delivery instrument includes an outer catheter member attached to the expandable frame adjacent the proximal end and an inner catheter member attached to the expandable frame adjacent the distal end, wherein the inner catheter member is retracted into the outer catheter member to pivot each of the plurality of anchors to an inverted position in the collapsed anchor configuration.

Example 17 is the system of Example 16, wherein the delivery instrument is configured to release each of the plurality of anchors from the inverted position to the expanded anchor configuration.

Example 18 is the system of Example 16, wherein the inner catheter member is retracted into the outer catheter member to pivot each of the plurality of anchors outward and progressively downward to the inverted position.

Example 19 is the system of Example 16, wherein the inner catheter member is retracted into the outer catheter member to pivot each of the plurality of anchors from a foot angle of from 0 to 45 degrees to a foot angle of from 45 to 180 degrees.

Example 20 is the system of Example 16, wherein the inner catheter member is extended out of the outer catheter member to pivot each of the plurality of anchors to the expanded anchor configuration

While multiple embodiments are disclosed, still other embodiments of the present disclosure will become apparent to those skilled in the art from the following detailed description, which shows and describes illustrative embodiments of the disclosure. Accordingly, the drawings and detailed description are to be regarded as illustrative in nature and not restrictive.

### BRIEF DESCRIPTION OF THE DRAWINGS

Fig. 1A is a diagram illustrating a top (atrial) view of a heart valve prosthesis configured to be deployed within a native heart valve at a native heart valve annulus, in accordance with embodiments of the subject matter of the disclosure.
Fig. 1B is a diagram illustrating an anterior view of the heart valve prosthesis, in accordance with embodiments of the subject matter of the disclosure.
Fig. 2 is a diagram illustrating a prosthetic mitral valve annulus and anchor locations for the feet of the anchors disposed about the circumference of the prosthetic mitral valve annulus, in accordance with embodiments of the subject matter of the disclosure.
Fig. 3A is a schematic diagram illustrating a mitral valve having an annulus, a transition region below the annulus, and LV muscle below the transition region.
Fig 3B is a diagram illustrating tissue at the mitral valve, including the annulus, the transition region below the annulus, and the LV muscle situated below the transition region.
Fig. 4 is a diagram illustrating portions of a heart valve prosthesis, in accordance with embodiments of the subject matter of the disclosure.
Fig. 5A is a diagram illustrating a 30 degree foot angle of an anchor and foot with respect to the longitudinal axis of the valve, in accordance with embodiments of the subject matter of the disclosure.
Fig. 5B is a diagram illustrating a 0 degree foot angle of an anchor and foot with respect to the longitudinal axis of the valve, in accordance with embodiments of the subject matter of the disclosure.
Fig. 6 is a diagram illustrating embodiments of the profile of a foot, in accordance with embodiments of the subject matter of the disclosure.
Fig. 7 is a diagram illustrating an anchor and a foot having one of the diamond-like structures as depicted in iterations C-E (shown in Fig. 6), in accordance with embodiments of the subject matter of the disclosure.
Fig. 8 is a diagram illustrating a native mitral valve and multiple anchor locations for the feet of a heart valve prosthesis around the circumference of the native mitral valve, in accordance with embodiments of the subject matter of the disclosure.
Fig. 9 is a diagram illustrating the delivery system attached to the prosthetic heart valve in the expanded configuration with upward facing anchoring projections having 0 to 45 degree foot angles, in accordance with embodiments of the subject matter of the disclosure.
Fig. 10 is a diagram illustrating the delivery system collapsing the prosthetic heart valve, in accordance with embodiments of the subject matter of the disclosure.
Fig. 11 is a diagram illustrating the delivery system further collapsing the prosthetic heart valve, in accordance with embodiments of the subject matter of the disclosure.
Fig. 12 is a diagram illustrating the delivery system further collapsing the prosthetic heart valve, in accordance with embodiments of the subject matter of the disclosure.
Fig. 13 is a diagram illustrating the delivery system further collapsing the prosthetic heart valve, in accordance with embodiments of the subject matter of the disclosure.
Fig. 14 is a diagram illustrating the delivery system further collapsing the prosthetic heart valve, in accordance with embodiments of the subject matter of the disclosure.
Fig. 15 is a diagram illustrating the inverted state of the prosthetic heart valve in the collapsed configuration and the outer catheter member steered and oriented and advanced towards the native mitral valve annulus, in accordance with embodiments of the subject matter of the disclosure.
Fig. 16 is a diagram illustrating the repositioning of the anchoring projections and the feet or the removing of the prosthetic heart valve, in accordance with embodiments of the subject matter of the disclosure.

### DETAILED DESCRIPTION

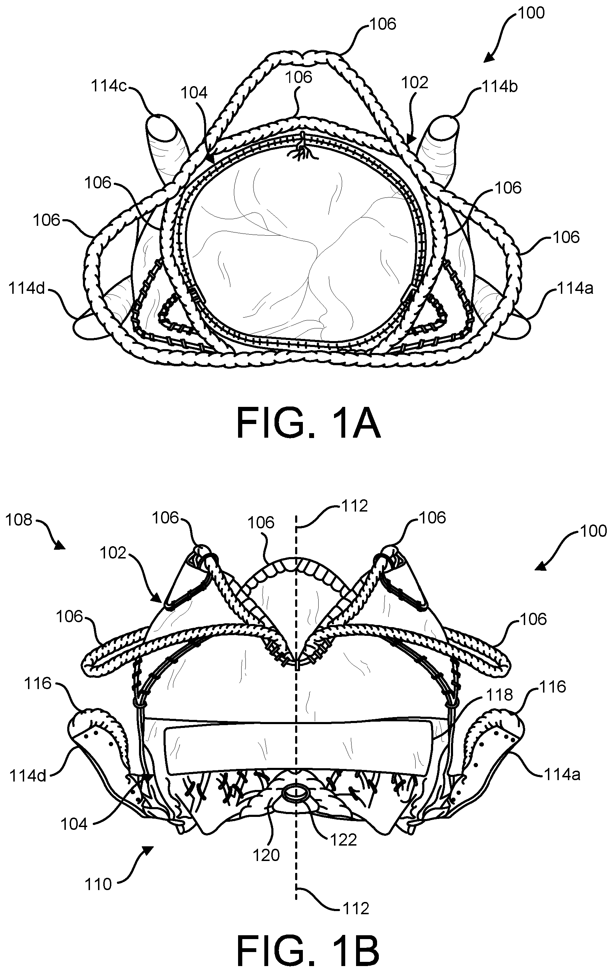

Fig. 1A is a diagram illustrating a top (atrial) view of a heart valve prosthesis 100 configured to be deployed within a native heart valve at a native heart valve annulus, in accordance with embodiments of the subject matter of the disclosure. Fig. 1B is a diagram illustrating an anterior view of the heart valve prosthesis 100, in accordance with embodiments of the subject matter of the disclosure. Figs. 1A and 1B illustrate the heart valve prosthesis 100 in an expanded configuration, as opposed to a collapsed configuration that is used for delivery of the heart valve prosthesis 100 to the native heart valve annulus.

The prosthesis 100 includes an anchor assembly 102 and a valve assembly 104. In some embodiments, the occluding function of the prosthesis 100 can be performed using configurations other than the depicted tri-leaflet occluder. For example, bi-leaflet, quad-leaflet, or mechanical valve constructs can be used in some embodiments.

The anchor assembly 102 includes an expandable frame 106 having a proximal end 108 and a distal end 110 with a longitudinal axis 112 extending therethrough. The expandable frame 106 is configured to collapse radially for delivery and expand radially upon deployment to the expanded configuration.

The anchor assembly 102 includes a plurality of spaced anchors 114a-114d extending from the distal end 110 of the expandable frame 106 towards the proximal end 108. Each of the anchors 114a-114d includes a free end or foot 116. Figs. 1A and 1B illustrate the anchors 114a-114d in an expanded anchor configuration for engaging subannular tissue (below the native heart valve annulus). Also, each of the anchors 114a-114d is expandable from a collapsed anchor configuration where the anchors 114a-114d are inverted such that the anchors 114a-114d point distally or downward in the collapsed anchor configuration.

As shown in Fig. 1B, a supplemental covering portion 118 can be positioned on an anterior surface of the valve assembly 104. The supplemental covering portion 118 can provide an enhanced sealing capability between the valve prosthesis 100 and surrounding native tissues. The supplemental covering portion 118 can be made of a material such as, but not limited to, DACRON^{®}, felt, polyester, a silicone, a urethane, ELAST-EON^{™} (a silicone and urethane polymer), another biocompatible polymer, polyethylene terephthalate (PET), copolymers, or combinations and subcombinations thereof. In embodiments, the valve prosthesis 100 also includes a systolic anterior motion (SAM) containment member 120 with an eyelet 122 for engaging and moving the SAM containment member 120.

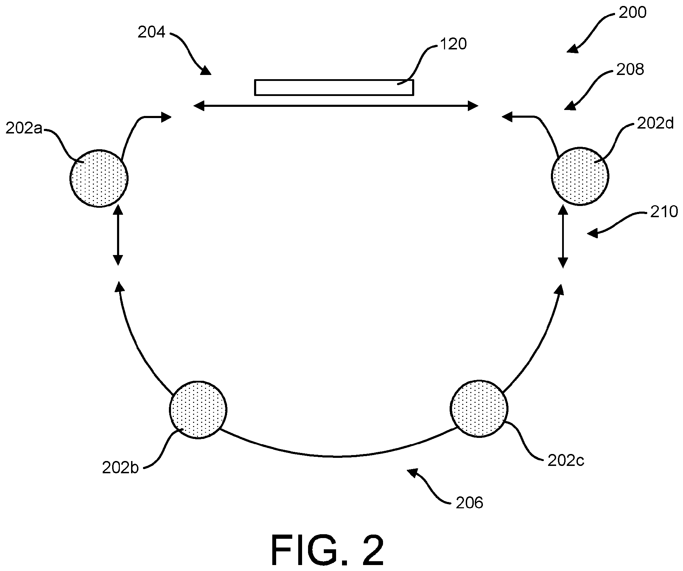

Fig. 2 is a diagram illustrating a prosthetic mitral valve annulus 200 and anchor locations 202a-202d for the feet 116 of the anchors 114a-114d disposed about the circumference of the prosthetic mitral valve annulus 200, in accordance with embodiments of the subject matter of the disclosure. The prosthetic mitral valve annulus 200 includes an anterior region 204, a posterior region 206, a commissural region 208, and a medial/lateral region 210. In embodiments, the prosthetic mitral valve 100 includes a SAM containment member 120 at the anterior region 204.

The heart valve prosthesis 100 includes four anchors 114a-114d, such that two of the anchors 114a and 114d are generally disposed at the anchor locations 202a and 202d, respectively, in the anterior region 204 and two of the anchors 114b and 114c are generally disposed at the anchor locations 202b and 202c, respectively, in the posterior region 206. In embodiments, the heart valve prosthesis 100 can include three anchors, two of which are generally disposed in the anterior region 204 and one of which is disposed in a generally central location of the posterior region 206. In other embodiments, the heart valve prosthesis 100 can include more than three or four anchors.

In some embodiments, the heart valve prosthesis 100 design and configuration are any one of the prosthesis designs and configurations disclosed in United States Patent Application Publication No. 2017/0189177 or United States Patent Application Publication No. 2019/0029814. While the concepts disclosed herein may be used in conjunction with any heart valve, the following disclosure provides embodiments for a mitral valve prosthesis.

While the anchor locations 202a-202d are illustrated in certain locations around the circumference of the prosthetic mitral valve annulus 200 in Fig. 2, in embodiments, one or more of these locations can be adjusted, such as up to 10 degrees (clockwise or counterclockwise), about the circumference, i.e., perimeter, of the annulus 200. In some embodiments, the anchor locations 202a-202d are disposed at a circumferential location about the annulus 200, such that the anchor locations 202a-202d are generally aligned with native valve commissures. In this way, the interference of the anchors 114a-114d with the operation of the native leaflets is minimized.

By disposing the anchors 114a-114d at appropriate locations about the circumference of the annulus 200, the heart valve prosthesis 100 is adequately anchored such that during diastole, when the left ventricle contracts and the blood pressure drives the valve prosthesis toward the left atrium, the anchors 114a-114d contact subannular tissue, i.e., tissue below the annulus of the native heart valve, and thereby anchor the prosthesis 100 at the mitral valve annulus location.

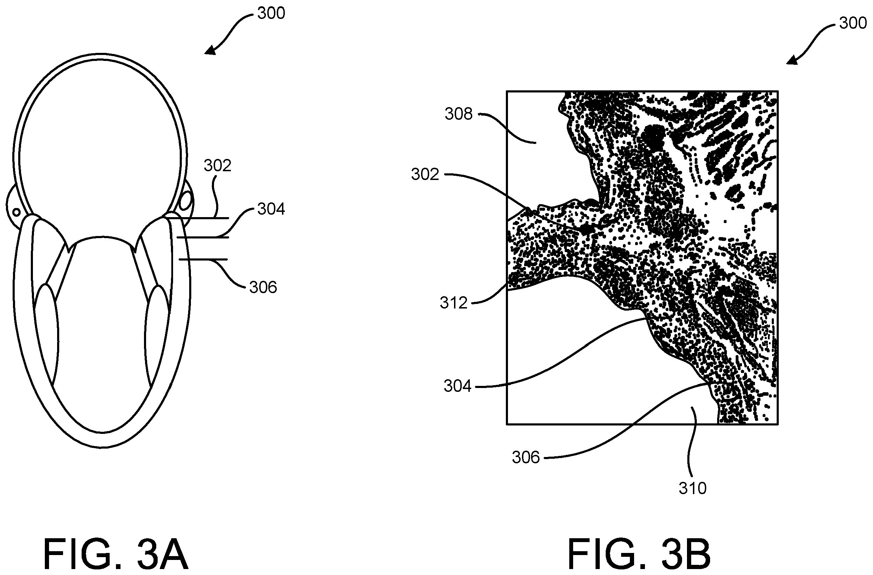

Fig. 3A is a schematic diagram illustrating a mitral valve 300 having an annulus 302, a transition region 304 below the annulus 302, and LV muscle 306 below the transition region 304. The location and size of these regions or areas may vary slightly from patient to patient. For example, in embodiments, the transition region 304 begins 1 to 3 millimeters (mm) below the annulus 302 and the LV muscle 306 begins 6 to 8 mm below the annulus 302, and, in embodiments, the transition region 304 ends where the LV muscle 306 begins. In embodiments, the transition region 304 is about 2 millimeters (mm) below the annulus 302 and the LV muscle 306 is about 7 mm below the annulus 302.

Fig 3B is a diagram illustrating tissue at the mitral valve 300, including the annulus 302, the transition region 304 below the annulus 302, and the LV muscle 306 situated below the transition region 304. As shown, the annulus 302 is situated between the left atrium 308 and the left ventricle 310, and a leaflet 312 branches from the annulus 302.

The annulus 302 is made up of fibrous tissue, such as collagen and/or reticular fibers, which have significantly high puncture resistance. The LV muscle substrate 306 is made up of cardiac muscle cells that have a somewhat lower puncture resistance as compared to the annulus 302. In embodiments, the prosthetic valve anchors 114a-114d load to the tissue of the annulus 302, the LV muscle 306, or the transition region 304.

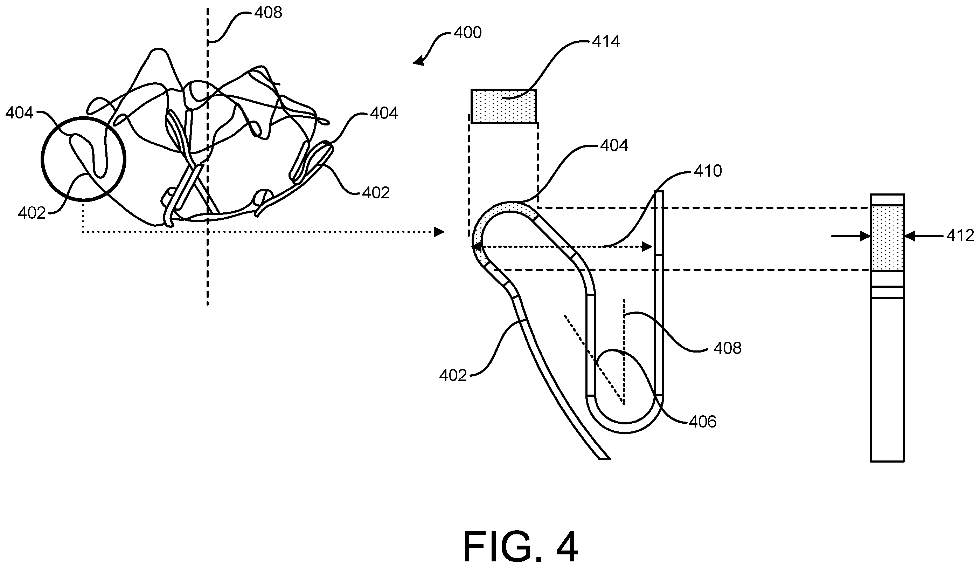

Fig. 4 is a diagram illustrating portions of a heart valve prosthesis 400, in accordance with embodiments of the subject matter of the disclosure. As shown, the prosthesis 400 includes anchors 402 with feet 404 for contacting tissue adjacent the native heart valve. In embodiments, the prosthesis 400 is like the heart valve prosthesis 100 of Figs. 1A and 1B. Also, in embodiments, the anchors 402 and feet 404 are like the anchors 114a-114d and feet 116 (shown in Figs. 1A and 1B).

The anchors 402 and the feet 404 are configured to contact the subannular tissue on the ventricular side of the valve annulus. As shown in Fig. 4, the foot 404 is configured with a "foot angle" 406 defined as the angle of the foot 404 with respect to the longitudinal axis 408 of the heart valve prosthesis 400 and a "toe out distance" 410, which is defined as the distance the foot 404 extends radially outwardly from the valve body. Additionally, the foot 404 includes a certain foot width 412 traveling through a certain arc length, which collectively defines a foot contact surface area, indicated at 414. By adjusting these parameters, the foot contact surface area at 414 may be adjusted to an appropriate level to properly support the forces generated during the heart cycle. For example, by increasing the foot contact surface area at 414, the pressure on the tissue adjacent the annulus may be reduced.

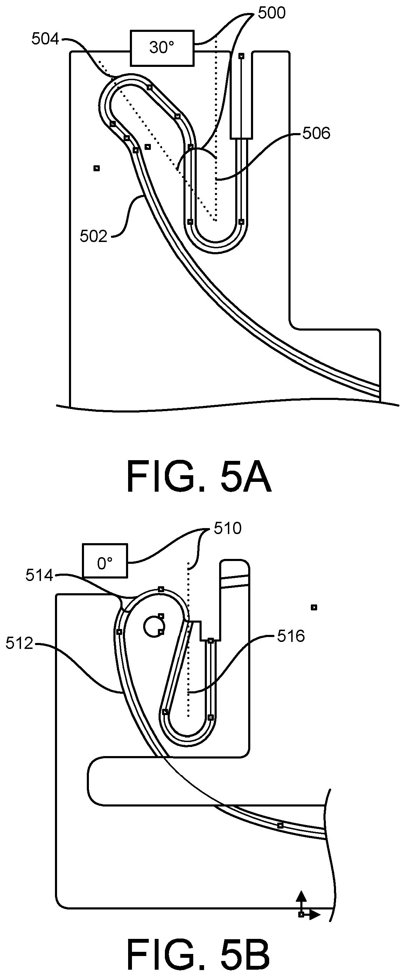

Figs. 5A and 5B are diagrams illustrating various foot angles of anchors and feet, in accordance with embodiments of the subject matter of the disclosure. The foot angles in Figs. 5A and 5B are defined as the angle of the foot with respect to the longitudinal axis of the heart valve prosthesis, as described above.

Fig. 5A is a diagram illustrating a 30 degree foot angle 500 of anchor 502 and foot 504 with respect to the longitudinal axis, indicated at 506, of the valve, in accordance with embodiments of the subject matter of the disclosure.

Fig. 5B is a diagram illustrating a 0 degree foot angle 510 of anchor 512 and foot 514 with respect to the longitudinal axis, indicated at 516, of the valve, in accordance with embodiments of the subject matter of the disclosure. Where, the anchor 512 and foot 514 are substantially parallel to the longitudinal axis 516 of the valve. In embodiments, the foot geometry is configured such that the foot angle is within the range of 0 to 45 degrees with respect to the longitudinal axis of the valve. In embodiments, the 0 degree foot angle 510 aligns the foot 514 with the fibrous annulus tissue of the heart valve.

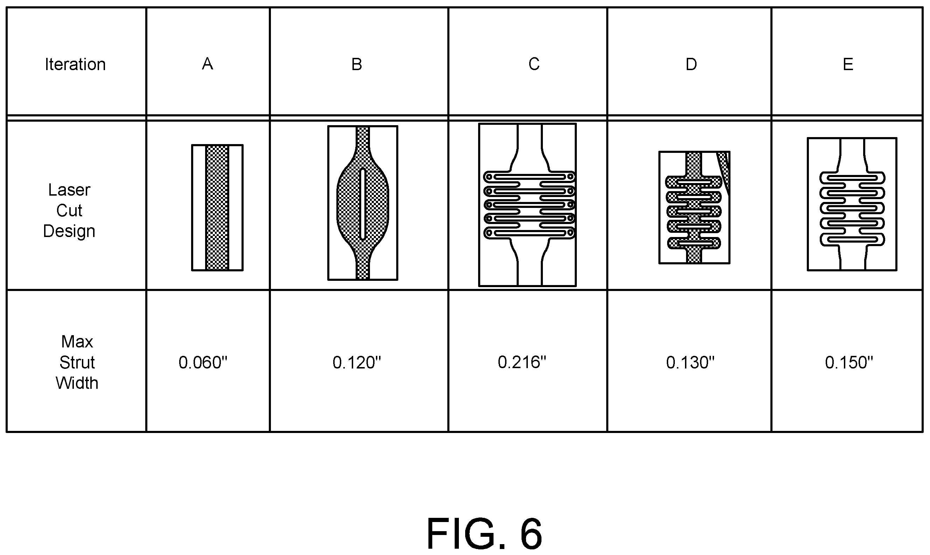

In some embodiments, the foot geometry is designed to ensure that the foot contact surface area 414 is such that the maximum pressure exerted by the foot is less than the puncture resistance of the substrate, i.e., the native heart valve tissue contacted by the foot of the prosthesis. Where, the foot geometry and the foot contact surface area 414 are based on multiple items, such as the foot angle, the arc length of the foot, the arc radius of the foot, and the foot width, which can be adjusted to ensure that the maximum pressure exerted by the foot is less than the puncture resistance of the substrate. In addition, the contact surface area 414 can be adjusted or controlled by modifying the profile of the foot at the contact location, as illustrated in Fig. 6, which may be a laser cut profile.

Fig. 6 is a diagram illustrating embodiments of the profile of a foot, such as the feet 404, 504, and 514, in accordance with embodiments of the subject matter of the disclosure. As illustrated, iteration A of the foot has a straight width with a maximum width of .06 inches, iteration B has a hexagonal shaped, diamond-like structure with a maximum width of .12 inches (1 inch is equal to 2.54cm), iteration C has multiple diamond-like structures with a maximum width of .216 inches, iteration D has multiple diamond-like structures with a maximum width of .13 inches, and iteration E has multiple diamond-like structures with a maximum width of .15 inches.

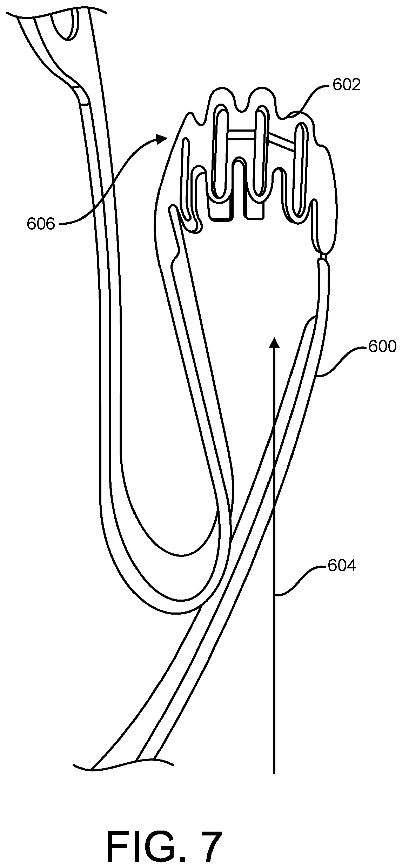

Fig. 7 is a diagram illustrating an anchor 600 and a foot 602 having one of the diamond-like structures as depicted in iterations C-E (shown in Fig. 6), in accordance with embodiments of the subject matter of the disclosure. The anchor 600 and the foot 602 are at a foot angle 604 of 0 degrees and the multiple diamond-like structures on the foot 602 provide an increased loading or contact surface area 606.

Also, addition of the diamond-like structures in iterations B-E, as compared to only increasing the strut width, allows for easier formability and manufacturability of these parts as well as improved deliverability.

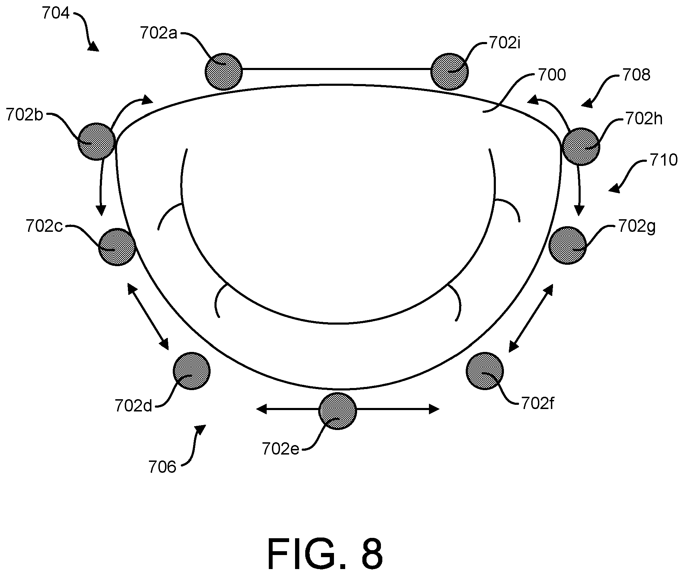

Fig. 8 is a diagram illustrating a native mitral valve 700 and multiple anchor locations 702a-702i for the feet of a heart valve prosthesis around the circumference of the native mitral valve 700, in accordance with embodiments of the subject matter of the disclosure. The native mitral valve 700 includes an anterior region 704, a posterior region 706, a commissural region 708, and a medial/lateral region 710.

By adjusting variables including one or more of the locations of the anchors, the number of anchors, the number of feet, the number of feet per anchor, foot angles, foot widths, arc length, and arc radius, the transfer forces and puncture pressures generated by the prosthetic valve may be increased or decreased. Where, in embodiments, a primary function of the foot is to provide stable anchoring to the native heart valve without puncturing into the loading tissue, i.e., the substrate, of the heart. Also, in embodiments, the optimized foot locations in combination with the foot geometry ensure that the foot does not puncture into or through the substrate.

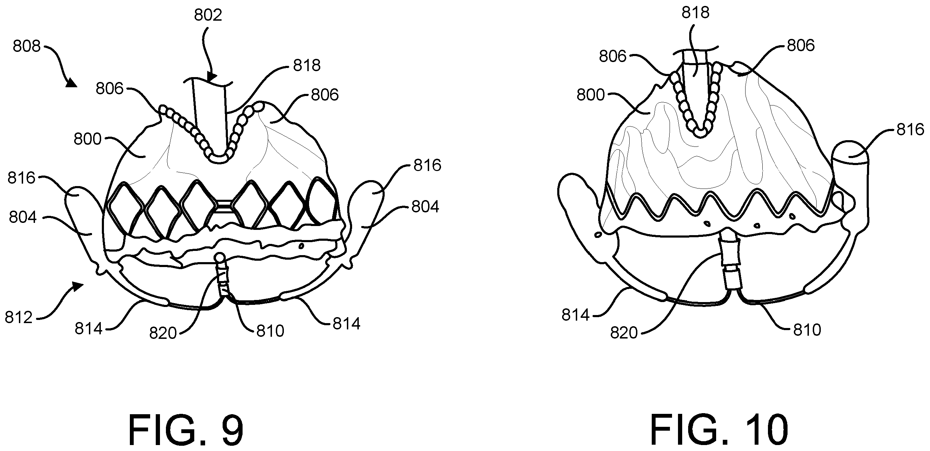

Figs. 9-14 are diagrams illustrating a mitral valve anchoring dock or replacement system that includes a prosthetic heart valve 800 and a delivery system or instrument 802, in accordance with embodiments of the subject matter of the disclosure. The prosthetic heart valve 800 and the delivery system 802 facilitate an inversion geometry to improve delivery and repositioning/removal of the prosthetic heart valve 800 to and from the native heart valve.

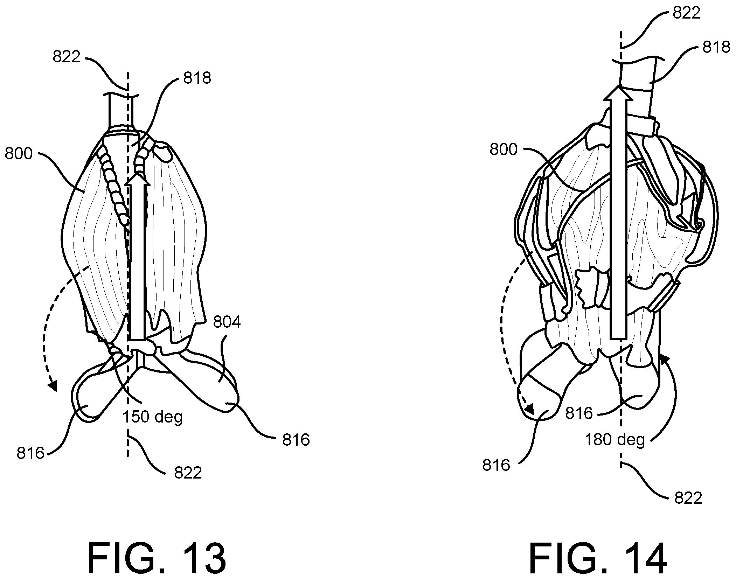

In Figs. 9-14, the prosthetic heart valve 800 is shown moving from an expanded configuration in Fig. 9 through various stages to a collapsed configuration in Fig. 14, where the prosthetic heart valve 800 can be delivered to the native annulus. While Figs. 9-14 and the accompanying text relate to a certain prosthetic heart valve design, the same general delivery technique may be used with any of a variety of other designs that include anchoring feet, including those designs disclosed in the United States Patent Publications cited above.

Fig. 9 is a diagram illustrating the delivery system 802 attached to the prosthetic heart valve 800, i.e., the implant, in the expanded configuration with upward facing anchoring projections 804 having 0 to 45 degree foot angles, in accordance with embodiments of the subject matter of the disclosure. The prosthetic heart valve 800 includes implant arches 806 at the proximal end 808 of the prosthetic heart valve 800, a hub 810 at the distal end 812 of the prosthetic heart valve 800, and elongated LV frame members 814 that extend from the hub 810 to the anchoring projections 804 and feet 816 on the anchoring projections 804.

The delivery system 802 includes an outer catheter member 818 attached to the proximal end 808 of the prosthetic heart valve 800 adjacent the heart valve implant arches 806 and an inner catheter member 820 attached to the hub 810 at the distal end 812 of the prosthetic heart valve 800. In this configuration, the anchoring projections 804 including the feet 816 point upward. In embodiments, the number of anchoring projections 804 and/or elongated LV frame members 814 vary from two to nine. The elongated LV frame member's length, stiffness, angle relative to the feet 816 and the hub 810, and curvature facilitate the inversion angle of the feet 816. In embodiments, the delivery system 802 facilitates inversion of the prosthetic heart valve 800 through a flexible inner catheter member 820 at the hub 810 and a rigid outer catheter member 818 at the implant arches 806. In embodiments, the outer catheter member 818 is connected to the prosthetic heart valve 800 at other frame locations to further facilitate the inversion geometry.

Fig. 10 is a diagram illustrating the delivery system 802 collapsing the prosthetic heart valve 800, in accordance with embodiments of the subject matter of the disclosure. To collapse the prosthetic heart valve 800, the inner catheter member 820 is retracted into the outer catheter member 818 and the implant arches 806 collapse against the outer catheter 818. The inner catheter member 820 attached to the hub 810 is retracted upward through a central opening in the outer catheter member 818 to contain the elongated LV frame members 814 of the prosthetic heart valve 800 within the outer catheter member 818.

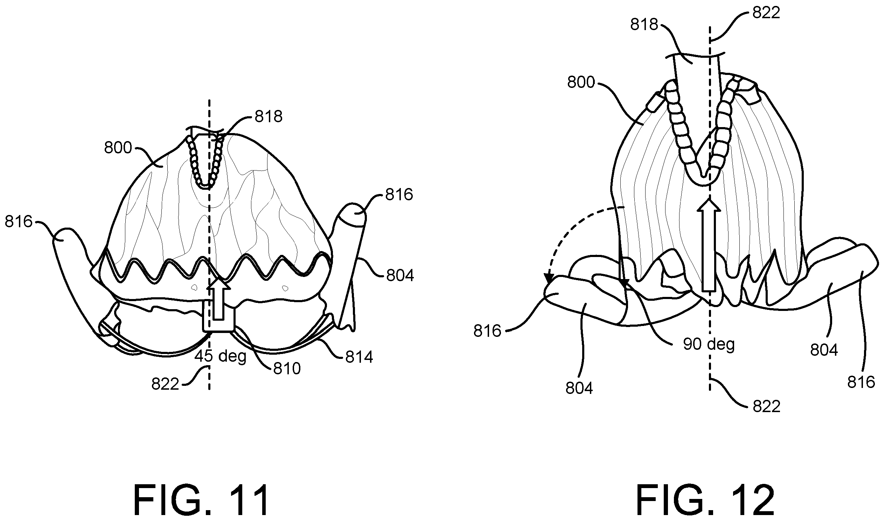

Fig. 11 is a diagram illustrating the delivery system 802 further collapsing the prosthetic heart valve 800, in accordance with embodiments of the subject matter of the disclosure. As shown in Fig. 11, the inner catheter member 820 is further retracted into the outer catheter member 818 and the elongated LV frame members 814 are further retracted into the outer catheter 818. Also, the anchoring projections 804 and the feet 816 pivot outward and progressively downward as the inner catheter member 820 is retracted into the outer catheter 818. In Fig. 11, the anchoring projections 804 and the feet 816 are at a foot angle of about 45 degrees from the longitudinal axis 822 of the prosthetic heart valve 800.

Fig. 12 is a diagram illustrating the delivery system 802 further collapsing the prosthetic heart valve 800, in accordance with embodiments of the subject matter of the disclosure. As shown in Fig. 12, the inner catheter member 820 is further retracted into the outer catheter 818, the elongated LV frame members 814 are further retracted into the outer catheter 818, and the anchoring projections 804 and the feet 816 further pivot outward and progressively downward as the inner catheter member 820 is retracted into the outer catheter 818. In Fig. 12, the anchoring projections 804 and the feet 816 are at a foot angle of about 90 degrees from the longitudinal axis 822 of the prosthetic heart valve 800.

Fig. 13 is a diagram illustrating the delivery system 802 further collapsing the prosthetic heart valve 800, in accordance with embodiments of the subject matter of the disclosure. As shown in Fig. 13, the inner catheter member 820 is further retracted into the outer catheter 818, the elongated LV frame members 814 are further retracted into the outer catheter 818, and the anchoring projections 804 and the feet 816 further pivot outward and progressively downward as the inner catheter member 820 is retracted into the outer catheter 818. In Fig. 13, the anchoring projections 804 and the feet 816 are at a foot angle of about 150 degrees from the longitudinal axis 822 of the prosthetic heart valve 800.

Fig. 14 is a diagram illustrating the delivery system 802 further collapsing the prosthetic heart valve 800, in accordance with embodiments of the subject matter of the disclosure. As shown in Fig. 14, the inner catheter member 820 is further retracted into the outer catheter 818, the elongated LV frame members 814 are further retracted into the outer catheter 818, and the anchoring projections 804 and the feet 816 further pivot outward and progressively downward as the inner catheter member 820 is retracted into the outer catheter 818. In Fig. 14, the anchoring projections 804 and the feet 816 are at a foot angle of about 180 degrees from the longitudinal axis 822 of the prosthetic heart valve 800.

Thus, the anchoring projections 804 and the feet 816 pivot outward and progressively downward from the expanded configuration foot angles of 0 to 45 degrees to the collapsed configuration foot angles of between 45 and 180 degrees as the inner catheter member 820 is retracted into the outer catheter 818. Once the desired inverted delivery geometry is achieved the inner catheter member 820 is locked in position relative to the outer catheter member 818.

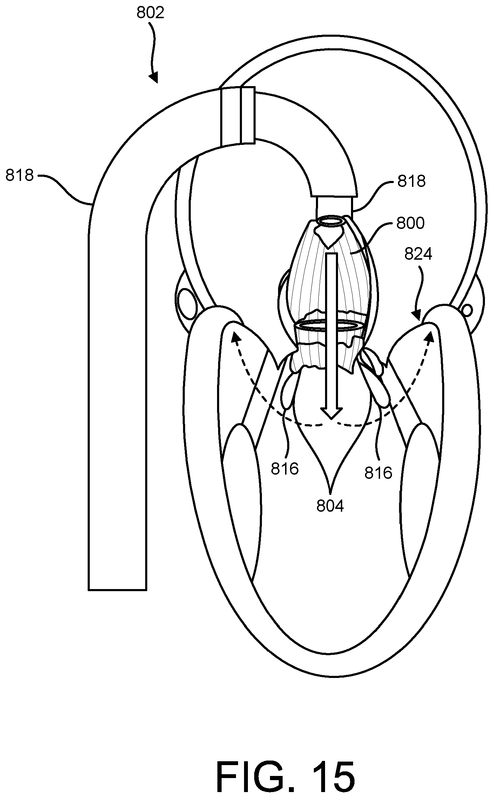

Fig. 15 is a diagram illustrating the inverted state of the prosthetic heart valve 800 in the collapsed configuration and the outer catheter member 818 steered and oriented using other members of the delivery system (not described here) and advanced towards the native mitral valve annulus 824, in accordance with embodiments of the subject matter of the disclosure. In delivery or readjustment, once the tips of the anchoring projections 804, i.e., the feet 816, are sufficiently past the annulus and in good orientation, the inner catheter member 820 is unlocked relative to the outer catheter member 818 and gradually advanced downward, as indicated by the arrow in Fig. 15. This releases the hub 810 and the elongated LV frame members 814 of the prosthetic heart valve 800 downward and out of the outer catheter member 818, which allows the anchoring projections 814 to deploy radially, repositioning until they reach the upward facing expanded configuration and foot angles of 0 to 45 degrees, where they remain post deployment. In this expanded, deployed configuration, the anchoring projections 804 and the feet 816 contact subannular tissue to adequately anchor the prosthetic heart valve 800 at the mitral valve annulus 824.

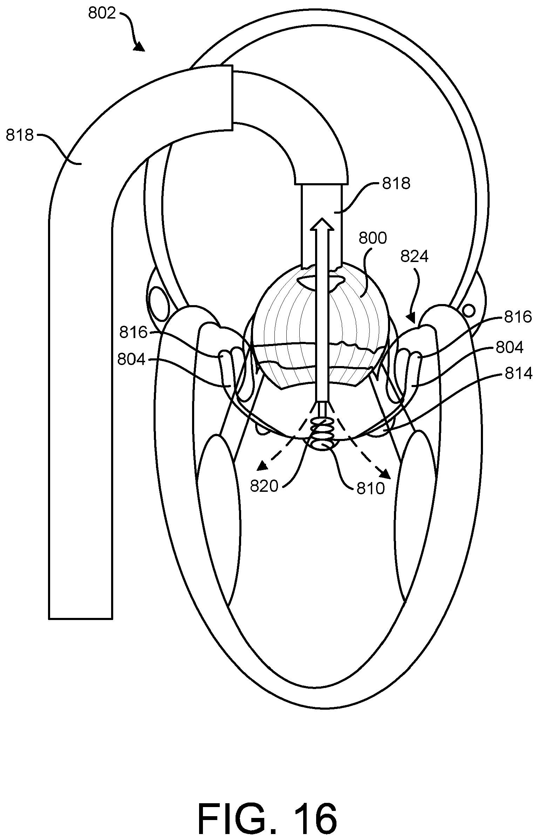

Fig. 16 is a diagram illustrating the repositioning of the anchoring projections 804 and the feet 816 or the removing of the prosthetic heart valve 800, where the deployment process described above is reversed, in accordance with embodiments of the subject matter of the disclosure. The inner catheter member 820 attached to the hub 810 is retracted upward through the central opening in the outer catheter member 818, such as to contain the elongated LV frame members 814 within the outer catheter member 818. As the elongated LV frame members 814 move inward and into the outer catheter member 818, the anchoring projections 804 and the feet 816 pivot to an outward and progressively downward position releasing them from the native structures. The outer catheter member 818 can be advanced or retracted further to remove the prosthetic heart valve 800 from the native mitral valve for removal and/or to attempt redelivery.

This inverted delivery technique allows for a prosthetic heart valve 800 having a 0 degree foot angle, i.e., a foot oriented generally parallel to the longitudinal axis of the prosthetic heart valve 800, to be easily delivered. Further, the inverted delivery technique enables reduced delivery depth since the feet 816 are facing forward and only the feet 816 cross the native valve annulus as opposed to each of the hub 810, the elongated LV frame member 814, and the implant arches 306 or other components of the prosthetic heart valve 800. This inverted technique also allows for improved repositioning as the prosthetic heart valve 800 can be retracted into the delivery catheter system 802 to invert back the feet 816 and redeliver the prosthetic heart valve 800. Further, this technique allows for a reduction in imaging intensity.

## Claims

1. A valve prosthesis system, comprising:
a prosthetic valve (100, 800) having an expandable frame (106) including a proximal end (108) and a distal end (110) and a longitudinal axis (112) extending therethrough, the expandable frame configured to collapse radially for delivery and expand radially upon deployment to an expanded configuration, the prosthetic valve including a plurality of anchors (114a-114d), each anchor being expandable from a collapsed anchor configuration to an expanded anchor configuration;
**characterized by**
a delivery instrument (802) that includes an outer catheter member (818) attached to the expandable frame adjacent the proximal end and an inner catheter member (820) attached to the expandable frame adjacent the distal end,
wherein the plurality of anchors (114a-114d) extending from the distal end (110) of the expandable frame (106) towards the proximal end (108); and
wherein the inner catheter member (820) is retracted into the outer catheter member (818) to pivot each of the plurality of anchors (114a-114d) to an inverted position in the collapsed anchor configuration.

2. The system of claim 1, wherein the delivery instrument is configured to release each of the plurality of anchors from the inverted position to the expanded anchor configuration.

3. The system of claim 1 or 2, wherein the inner catheter member is retracted into the outer catheter member to pivot each of the plurality of anchors outward and progressively downward to the inverted position.

4. The system of claim 1 or 2, wherein the inner catheter member is retracted into the outer catheter member to pivot each of the plurality of anchors from a foot angle of from 0 to 45 degrees to a foot angle of from 45 to 180 degrees.

5. The system of one of the preceding claims, wherein the inner catheter member is extended out of the outer catheter member to pivot each of the plurality of anchors to the expanded anchor configuration.

## Patentansprüche

1. Herzklappenprothesen-System das umfasst:
eine Herzklappenprothese (100, 800), die einen ausdehnbaren Rahmen (106) aufweist, der ein proximales Ende (108) sowie ein distales Ende (110) und eine Längsachse (112) einschließt, die sich durch diese hindurch erstreckt, wobei der ausdehnbare Rahmen so ausgeführt ist, dass er zum Einsetzen radial zusammengedrückt wird und sich bei Entfaltung in eine ausgedehnte Konfiguration radial ausdehnt, die Herzklappenprothese eine Vielzahl von Ankern (114a-114d) enthält und jeder Anker von einer zusammengedrückten Anker-Konfiguration in eine ausgedehnte Anker-Konfiguration ausgedehnt werden kann;
**gekennzeichnet durch**
ein Einsetz-Instrument (802), das ein äußeres Katheter-Element (818), das an dem ausdehnbaren Rahmen an das proximale Ende angrenzend angebracht ist, sowie ein inneres Katheter-Element (820) einschließt, das an dem ausdehnbaren Rahmen an das distale Ende angrenzend angebracht ist,
wobei die Vielzahl von Ankern (114a-114d) sich von dem distalen Ende (110) des ausdehnbaren Rahmens (106) auf das proximale Ende (108) zu erstreckt; und
wobei das innere Katheter-Element (820) in das äußere Katheter-Element (818) eingezogen wird und dabei jeden der Vielzahl von Ankern (114a-114d) an eine umgekehrte Position in der zusammengedrückten Anker-Konfiguration schwenkt.

2. System nach Anspruch 1, wobei das Einsetz-Instrument so ausgeführt ist, dass es jeden der Vielzahl von Ankern von der umgekehrten Position an die zusammengedrückte Anker-Konfiguration freigibt.

3. System nach Anspruch 1 oder 2, wobei das innere Katheter-Element in das äußere Katheter-Element eingezogen wird und dabei jeden der Vielzahl von Ankern nach außen und zunehmend nach unten an die umgekehrte Position schwenkt.

4. System nach Anspruch 1 oder 2, wobei das innere Katheter-Element in das äußere Katheter-Element eingezogen wird und dabei jeden der Vielzahl von Ankern von einem Fußwinkel von 0 bis 45° an einen Fußwinkel von 45 bis 180° schwenkt.

5. System nach einem der vorangehenden Ansprüche, wobei das innere Katheter-Element aus dem äußeren Katheter-Element heraus ausgefahren wird und dabei jeden der Vielzahl von Ankern an die ausgedehnte Anker-Konfiguration schwenkt.

## Revendications

1. Système de prothèse valvulaire, comprenant :
une valve prothétique (100, 800) comportant une structure extensible (106) incluant une extrémité proximale (108) et une extrémité distale (110) ainsi qu'un axe longitudinal (112) traversant, la structure extensible étant configurée pour se replier radialement afin d'être posée et étendue radialement lors d'un déploiement vers une configuration étendue, la valve prothétique incluant une pluralité d'ancrages (114a-114d), chaque ancrage étant extensible d'une configuration d'ancrage repliée à une configuration d'ancrage étendue ;
**caractérisé par**
un instrument de pose (802) qui inclut un élément de cathéter externe (818) attaché à la structure extensible adjacente à l'extrémité proximale et un élément de cathéter interne (820) attaché à la structure extensible adjacente à l'extrémité distale,
dans lequel la pluralité d'ancrages (114a-114d) s'étend depuis l'extrémité distale (110) de la structure extensible (106) vers l'extrémité proximale (108) ; et
dans lequel l'élément de cathéter interne (820) est rétracté dans l'élément de cathéter externe (818) pour faire pivoter chaque ancrage de la pluralité d'ancrages (114a-114d) vers une position inversée dans la configuration d'ancrage repliée.

2. Système selon la revendication 1, dans lequel l'instrument de pose est configuré pour libérer chaque ancrage de la pluralité d'ancrages de la position inversée à la configuration d'ancrage étendue.

3. Système selon la revendication 1 ou 2, dans lequel l'élément de cathéter interne est rétracté dans l'élément de cathéter externe pour faire pivoter chaque ancrage de la pluralité d'ancrages vers l'extérieur et progressivement vers le bas jusqu'à la position inversée.

4. Système selon la revendication 1 ou 2, dans lequel l'élément de cathéter interne est rétracté dans l'élément de cathéter externe pour faire pivoter chaque ancrage de la pluralité d'ancrages d'un angle de pied de 0 à 45 degrés à un angle de pied de 45 à 180 degrés.

5. Système selon l'une des revendications précédentes, dans lequel l'élément de cathéter interne est étendu hors de l'élément de cathéter externe pour faire pivoter chaque ancrage de la pluralité d'ancrages vers la configuration d'ancrage étendue.
